# EUROPEAN PATENT SPECIFICATION

(11) **EP 3 188 212 B1**
(45) Date of publication and mention of the grant of the patent: **01.08.2018**
(21) Application number: 16191333.0
(22) Date of filing: 16.11.2009
(51) Int. Cl.: H01J 21/10, H03F 3/44, H05G 1/22, A23L 3/26, A61L 2/00, A61L 11/00, B09C 1/00, C02F 1/00

(54) **METHOD OF ACTIVATING CASCADED ELECTRON TUBE STAGES WITHIN A COMMON VACUUM ENCLOSURE**
VERFAHREN ZUR AKTIVIERUNG VON KASKADIERTEN ELEKTRONENRÖHRENSTUFEN IN EINER GEMEINSAMEN VAKUUMHÜLLE
PROCÉDÉ D'ACTIVATION DES ÉTAGES CASCADES DE TUBE D'ÉLECTRON DANS UNE ENCEINTE À VID COMMUNE

(30) Priority: 18.05.2009 US 467974; 18.05.2009 WO PCT/US2009/044410
(43) Date of publication of application: 05.07.2017
(62) Divisional of application: 09845045.5
(73) Proprietor: Advanced Fusion Systems LLC, Newtown, CT 06470 (US)
(72) Inventor: BIRNBACH, Curtis A., New Rochelle, NY 10804 (US)
(74) Representative: Dennemeyer & Associates S.A.

(56) References cited:
- GB-A- 877 019
- US-A- 2 109 225
- US-A- 2 474 435
- US-A- 3 024 423
- US-A- 4 950 962
- US-A- 5 030 921
- Ed Kurtz: "Tube Maintenance Guide", , 1 January 2007 (2007-01-01), XP055211029, Retrieved from the Internet: URL:http://www.rell.com/filebase/en/src/Li terature/TubeMaintenanceGuide.pdf [retrieved on 2015-09-03]

## Description

### CROSS-REFERENCE TO RELATED APPLICATIONS

The application is a divisional application of European Patent Application 09845045.5, which is a national phase application of PCT/US2009/064619, filed November 16, 2009. The foregoing PCT application claims priority to (1) U.S. Application Serial No. 12/467,974 filed May 18, 2009 and (2) PCT/US2009/044410, filed May 18, 2009.

### FIELD OF THE INVENTION

The invention relates to a method of activating a plurality of electron tube stages with a cascaded structure in a common vacuum enclosure.

### BACKGROUND OF THE INVENTION

Activation of electron tubes is the process by which the cathode is converted from its as-manufactured state into a functioning electron emitter. Typically, this process involves drawing current from the cathode through the anode, while the tube is still connected to a vacuum pumping system. Specific implementation varies with the type of cathode used. Activation requires supplying operating voltages equal to or greater than those normally encountered in operation of the tube. Activation takes place while the tube is still connected to an external vacuum pump system. This is done to facilitate the removal of impurities released from the cathode by the activation process. In the case of very high voltage tubes, the cost of suitable power supplies is very high. It would, therefore, be desirable to minimize the cost of high voltage power supplies and to simplify and expedite the manufacturing process.

Any of GB 877,019, US 2,474,435 and US 3,024,423 discloses a plurality of cascaded electron tube states, in particular a plurality of cascaded electron tubes, interconnected in series, from a non-final stage to a final stage, in such a manner that in each non-final stage an electrode is connected to an electrode of a subsequent stage by a respective electrical interconnection. In US 5,030,921 a plurality of cascaded electron tube stages is disclosed, wherein the anode of each non-final stage is identical with the cathode of the subsequent stage.

### BRIEF SUMMARY OF THE INVENTION

The invention provides a method of activating a plurality of cascaded electron tube stages within a common vacuum enclosure. The method comprises interconnecting the plurality of cascaded electron tube stages in series, from a non-final stage to a final stage, in such a manner that in each non-final stage, an electrode is connected to an electrode of a subsequent stage by a respective electrical interconnection. At least one of said respective electrical interconnections comprises a linking structure for electrically and mechanically joining an electrode of a previous stage with an electrode of a subsequent stage. The linking structure is the primary mechanical support for said electrode of the previous stage and for said electrode of the subsequent stage; the electrode of the previous stage comprises an anode and the electrode of the subsequent stage comprises a cathode. The plurality of cascaded electron tube stages is placed within the common vacuum enclosure and air is exhausted from the enclosure. An electrical voltage is provided between cathode and anode, being the electrodes, of a first serially-connected stage of said cascaded electron tube stages, so as to supply electrical energy to the first stage. A sufficient amount of said energy serially propagates through any intervening stage to the final stage so as to facilitate activation of all tube stages.

The foregoing method avoids the drawbacks of the prior art method of activating individual tubes mentioned above. This is accomplished by using, in a preferred form, only a single power supply to activate all the stages cascaded electron tube stages nearly simultaneously. The power supply needs to only meet the voltage requirement of the first stage tube, since the increased voltage required for each succeeding stage is provided by the voltage gain of the preceding stage. This avoids the need for larger and more costly power supplies for the succeeding stages, and for larger and substantially more complex exhaust stations involving, for instance, the use of larger feedthroughs that then require a larger vacuum enclosure and increased vacuum pumping and heating requirements.

### BRIEF DESCRIPTION OF DRAWINGS

In the drawings, in which like reference numerals refer to like parts:
FIG. 1 is a simplified perspective view, partially cut away, of key parts of three cascaded electron tube stages within a common vacuum enclosure, with various parts omitted for clarity, which may be activated in accordance with the invention.
FIG. 2 is a block diagram view of a scheme for activating integrated cascaded electron tube stages within a common vacuum enclosure; and
FIG. 3 is a block diagram view of a variable number of stages of cascades electron tube stages within a common vacuum enclosure.

### DETAILED DESCRIPTION OF THE INVENTION

### Inventive Method of Activating Cascaded Electron Tube Stages within a Common Vacuum Enclosure

Activation of an electron tube is the penultimate process step in the manufacture of the tube, just prior to pinching off the exhaust tubulation. The purpose of activation is to convert the as-manufactured cathode into a functioning electron emission element. Typically, this process involves drawing current from the cathode through the anode, while the tube is still connected to a vacuum pumping system. Specific implementation varies with the type of cathode used. It is important to recognize that the activation process is completely independent of the electrode geometry of the tube or tube stage.

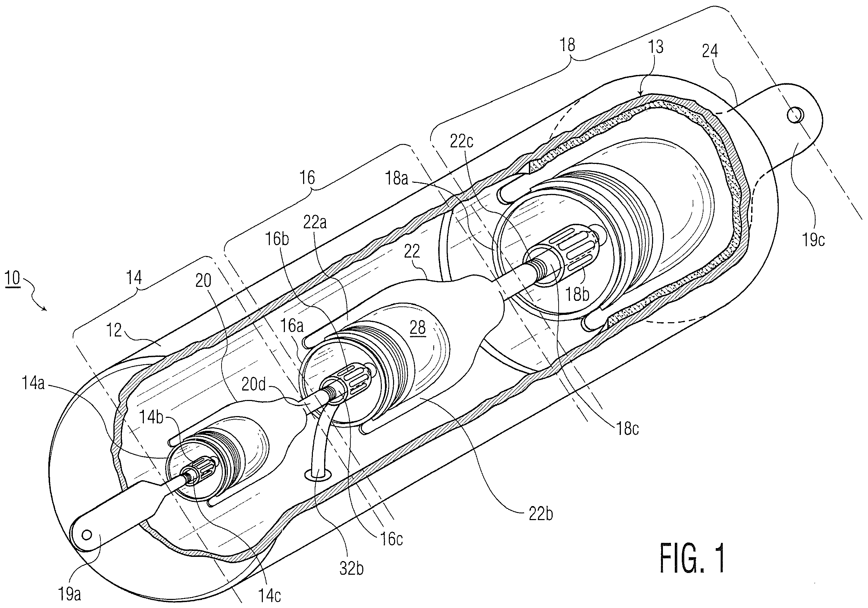

The present method of activating applies to the integrally series-connected electron tube stages within a common vacuum enclosure as described in connection with FIG. 1. Activation of a thermionic cathode primarily changes the chemistry of the emitting surface of the cathode, while activation of a cold cathode is used to remove impurities from the cathode.

FIG. 1 shows key parts of cascaded electron tube stages within a common vacuum enclosure 10 in accordance with an aspect of the invention. In FIG. 1, a vacuum enclosure 12 of nickel alloy, for instance, encloses non-final stages 14 and 16 and final stage 18 of cascaded electron tube stages 10. Cascaded electron tube stages 10 include first stage cathode input 19a, and last stage anode output 19c.

Non-final stage 16 includes an anode 16a, a grid 16b and a cathode 16c. A non-final linking structure 22 supports the anode 16a of non-final stage 16, as well as supports the cathode 18c of subsequent stage 18. The linking structure 22 generally has the form of a two-tined fork on one end, with tines 22a and 22b, a cathode support 22c, and an insulator 28. At one axial end of the linking structure 22 (along the axis of cathode 16c), linking structure 22 is connected to the anode 16a. The right-hand end of linking structure 22 terminates in a cathode support 22c for final stage 18.

FIG. 1 omits various elements for clarity, for instance, showing only grid feedthrough 32b. Further omitted for clarity from FIG. 1 are dielectric support elements for accurate positioning and supporting the various internal tube elements. Inclusion of such support elements will be routine to those of ordinary skill in the art.

The foregoing description of FIG. 1 has focused on the second non-final stage 16. The first stage 14 and the final stage 18 share much in common with the second stage 16, with some major differences as follows. Unlike the second stage 16, the first stage 14 has its cathode supported from a first stage cathode input 19a, rather than from a linking structure (e.g., akin to 20) from a prior stage. The final stage includes a linking structure 24 that terminates in a last stage anode output 19c, rather than a cathode support structure (e.g., 22c of second stage 16) for a following stage. The final stage 18 is electrically insulated from vacuum enclosure 12 by dielectric insulator 13. Finally, it should be noted that in this specification like reference numerals refer to like parts, so that, for instance, the foregoing description of anode 16a for second stage 16 applies as well to reference numeral 14a for first stage 14 and to 18a of third stage 18.

An aspect of the invention is the use of a single power supply to activate the successive stages 14, 16 and 18 (FIG. 1), as opposed to using three discrete power supplies of progressively increasing voltages. Since the cost of power high voltage power supplies increases rapidly with increasing output voltage rating, the ability to activate a very high voltage stage with a relatively low voltage power supply is desirable. This is achieved by taking advantage of the inherent amplification provided by each stage (14, 16 and 18). The first stage 14 raises the voltage to the correct level to properly activate the second stage 16; the same process is repeated for each successive stage. In the final stage (e.g., 18 or higher), the amplification process is still required but is used internally to activate that stage.

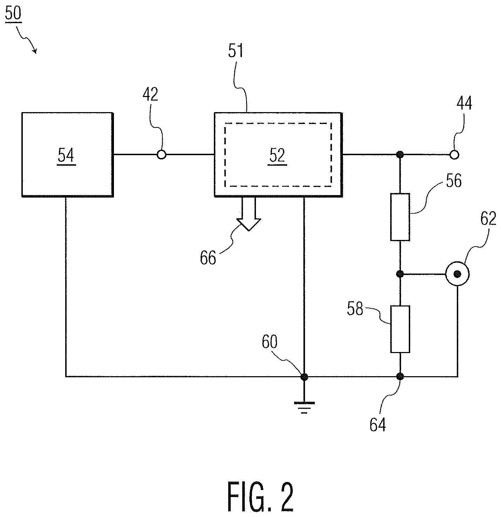

FIG. 2 shows a scheme 50 for activating integrated cascaded electron tube stages 52, shown in dashed lines within a vacuum enclosure 51, which may suitably comprise the cascaded electron tube stages described above in connection with FIG. 1. A variable high voltage power supply 54 feeds the input terminal 42 of amplifier 52. A load resistor 56 is connected to output terminal 44 on one end, and to a shunt resistor 58 on the other end. The other side of shunt resistor 58 is connected to common ground 60. The center conductor of a coaxial jack 62 is connected to the common terminal of load resistor 56 and shunt resistor 58. The ground connection of coaxial jack 62 is connected to the shunt resistor ground connection 64. An exhaust means 66, shown diagrammatically, is used to exhaust air and impurities from vacuum enclosure 51.

To activate the cascaded electron tubes 52, air is exhausted from enclosure 51 by exhaust means 66. Electrical voltage from variable high voltage power supply 54 is applied between the anode and cathode of the first serially connected serially connected tube stage within cascaded electron tube stages 52. A sufficient amount of the energy from power supply 54 is serially propagated through any intervening stage (here, the second stage) to the final stage so as to facilitate activation of all stages. Preferably, the energy supplied by the power supply 54 to the anode and cathode of the first stage is sufficient to cause activation of all stages. Beneficially, each stage amplifies the input activation voltage to the correct value for the level of activation mentioned in the preceding two sentences by virtue of its design.

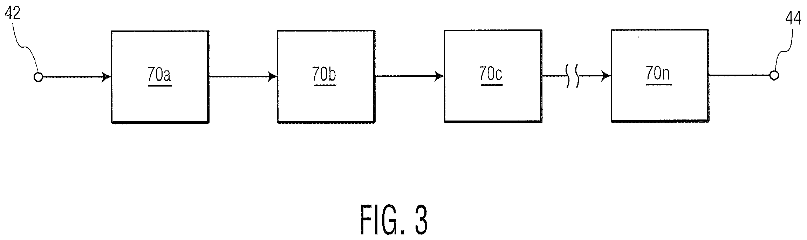

Although FIG. 2 and FIG. 1, show three stages of cascaded electron tube stages, four or more stages can be incorporated into cascaded electron tube stages. Thus, FIG. 3 diagrammatically shows, between input 42 and output 44, stages 70a, 70b, 70c and intervening, unnumbered stages, represented by a line break, until stage 70n. These four or more stages can replace the three stages of FIGS. 1 and 2. The interrelation of the various stages of FIG. 3 can be discerned from the interrelation of successive stages in FIG. 1. In particular, stage 70a (FIG. 3) corresponds to first stage 14 (FIG. 1), stages 70b, 70c and any further intervening stages in FIG. 3 correspond to second stage 16 in FIG. 3, and final stage 70n of FIG. 3 corresponding to final stage 18 in FIG. 1.

The scope of the invention should be not be limited by the preferred embodiments and examples, but should be given the broadest interpretation within the scope of the appended claims.

## Claims

1. A method of activating a plurality of cascaded electron tube stages (14, 16, 18) within a common vacuum enclosure (12), the method comprising:
a) interconnecting the plurality of cascaded electron tube stages (14, 16, 18) in series, from a non-final stage (14, 16) to a final stage (18), in such a manner that in each non-final stage an electrode (14a, 16a) is connected to an electrode (16c, 18c) of a subsequent stage by a respective electrical interconnection (20, 22);
b) at least one of said respective electrical interconnections comprising a linking structure (20, 22) for electrically and mechanically joining an electrode (14a, 16a) of a previous stage with an electrode (16c, 18c) of a subsequent stage; said linking structure (20, 22) being the primary mechanical support for said electrode (14a, 16a) of the previous stage and for said electrode (16c, 18c) of the subsequent stage, the electrode of the previous stage comprising an anode (16a, 18a) and the electrode of the subsequent stage comprising a cathode (16c, 18c);
c) placing the plurality of cascaded electron tube stages within the common vacuum enclosure (12) and exhausting air from the enclosure; and
d) providing electrical voltage between the cathode (16c) and the anode (14a), being the electrodes, of a first serially-connected stage of said cascaded electron tube stages, so as to supply electrical energy to the first stage; said energy serially propagating through any intervening stage to the final stage so as to facilitate activation of all tube stages.

2. The method of claim 2, wherein said electrical voltage between the cathode (18c) and the anode (16a) of the first serially-connected stage causes activation of all stages.

3. The method of claim 1, wherein each respective electrical interconnection comprises an electrical transmission line.

4. The method of claim 1, wherein the entire length of each linking structure forms an electrical transmission line.

5. The method of claim 1, wherein the step of interconnecting the plurality of cascaded electron tube stages includes providing cold cathode field emission electron tube stages as the electron tube stages.

6. The method of claim 1, wherein the plurality of cascaded electron tube stages is three in number.

7. The method of claim 1, wherein the plurality of cascaded electron tube stages is four in number.

## Patentansprüche

1. Verfahren zum Aktivieren einer Vielzahl von kaskadierten Elektronenröhrenstufen innerhalb einer gemeinsamen Vakuumhülle (12), wobei das Verfahren umfasst:
a) Verbinden der Vielzahl von kaskadierten Elektronenröhrenstufen in Reihe von einer Nicht-Endstufe (14, 16) zu einer Endstufe (18) derart, dass in jeder Nicht-Endstufe eine Elektrode (14a, 16a) mit einer Elektrode einer nachfolgenden Stufe durch eine jeweilige elektrische Verbindung (20, 22) verbunden wird;
b) wobei mindestens eine der jeweiligen elektrischen Verbindungen eine Verknüpfungsstruktur (20, 22) zum elektrischen und mechanischen Verbinden einer Elektrode (14a, 16a) einer vorhergehenden Stufe mit einer Elektrode (16c, 18c) einer nachfolgenden Stufe umfasst; wobei die Verknüpfungsstruktur (20, 22) der primäre mechanische Träger für die Elektrode (14a, 16a) der vorhergehenden Stufe und für die Elektrode (16c, 18c) der nachfolgenden Stufe ist, wobei die Elektrode der vorhergehenden Stufe eine Anode (16a, 18a) umfasst und die Elektrode der nachfolgenden Stufe eine Kathode (16c, 18c) umfasst;
c) Platzieren der Vielzahl von kaskadierten Elektronenröhrenstufen in der gemeinsamen Vakuumhülle (12) und Ablassen von Luft aus der Hülle; und
d) Bereitstellen einer elektrischen Spannung zwischen der Kathode (16c) und der Anode (14a) einer ersten in Reihe geschalteten Stufe, um der ersten Stufe elektrische Energie zuzuführen; wobei sich die Energie seriell durch beliebige dazwischenliegende Stufen bis zur Endstufe ausbreitet, um die Aktivierung aller Röhrenstufen zu erleichtern, wobei die Kathode (16c) und die Anode (14) die Elektroden sind.

2. Verfahren nach Anspruch 2, wobei die elektrische Spannung zwischen der Kathode (18c) und der Anode (16a) der ersten in Reihe geschalteten Stufe die Aktivierung aller Stufen bewirkt.

3. Verfahren nach Anspruch 1, wobei jede jeweilige elektrische Verbindung eine elektrische Übertragungsleitung umfasst.

4. Verfahren nach Anspruch 1, wobei die gesamte Länge jeder Verknüpfungsstruktur eine elektrische Übertragungsleitung bildet.

5. Verfahren nach Anspruch 1, wobei der Schritt des Verbindens der Vielzahl von kaskadierten Elektronenröhrenstufen das Bereitstellen von Kaltkathoden-Feldemissions-Elektronenröhrenstufen als Elektronenröhrenstufen beinhaltet.

6. Verfahren nach Anspruch 1, wobei die Vielzahl von kaskadierten Elektronenröhrenstufen drei beträgt.

7. Verfahren nach Anspruch 1, wobei die Vielzahl von kaskadierten Elektronenröhrenstufen vier beträgt.

## Revendications

1. Procédé d'activation d'une pluralité d'étages de tubes d'électrons en cascade dans une enceinte à vide (12) commune, le procédé comprenant :
a) l'interconnexion en série de la pluralité d'étages de tubes d'électrons en cascade, d'un étage non final (14, 16) jusqu'à un étage final (18), de sorte qu'à chaque étage non final, une électrode (14a, 16a) soit connectée à une électrode d'un étage suivant par une interconnexion électrique (20, 22) respective.
b) au moins l'une desdites interconnexions électriques respectives comprenant une structure de liaison (20, 22) pour joindre électriquement et mécaniquement une électrode (14a, 16a) d'un étage précédent à une électrode (16c, 18c) d'un étage suivant ; ladite structure de liaison (20, 22) étant le support mécanique principal pour ladite électrode (14a, 16a) de l'étage précédent et pour ladite électrode (16c, 18c) de l'étage suivant, l'électrode de l'étage précédent comprenant une anode (16a, 18a) et l'électrode de l'étage suivant comprenant une cathode (16c, 18c) ;
c) le placement de la pluralité d'étages de tubes d'électrons en cascade dans l'enceinte à vide (12) commune et l'évacuation de l'air de l'enceinte ; et
d) la fourniture d'une tension électrique entre la cathode (16c) et l'anode (14a) d'un premier étage connecté en série, de manière à fournir de l'énergie électrique au premier étage ; ladite énergie se propageant en série à travers n'importe quel étage intermédiaire jusqu'à l'étage final, de manière à faciliter l'activation de tous les étages de tubes, dans lequel la cathode (16c) et l'anode (14) sont les électrodes.

2. Procédé selon la revendication 2, dans lequel ladite tension électrique entre la cathode (18c) et l'anode (16a) du premier étage connecté en série provoque l'activation de tous les étages.

3. Procédé selon la revendication 1, dans lequel chaque interconnexion électrique respective comprend une ligne de transmission électrique.

4. Procédé selon la revendication 1, dans lequel la longueur totale de chaque structure de liaison forme une ligne de transmission électrique.

5. Procédé selon la revendication 1, dans lequel l'étape d'interconnexion de la pluralité d'étages de tubes d'électrons en cascade comprend la fourniture d'étages de tubes d'électrons à émission de champ à cathode froide en tant qu'étages de tubes d'électrons.

6. Procédé selon la revendication 1, dans lequel la pluralité d'étages de tubes d'électrons en cascade est au nombre de trois.

7. Procédé selon la revendication 1, dans lequel la pluralité d'étages de tubes d'électrons en cascade est au nombre de quatre.
